# FASCICULE DE BREVET EUROPEEN

(11) **EP 2 431 356 B1**
(45) Date de publication et mention de la délivrance du brevet: **13.11.2013**
(21) Numéro de dépôt: 11368025.0
(22) Date de dépôt: 16.09.2011
(51) Int. Cl.: A61K 8/64, C07C 323/60, A61Q 19/08

(54) **Peptidomimétiques dérivés de la méthionine et leur utilisation dans la protection des mitochondries des cellules cutanées**
Peptidomimetischen Derivaten vom Methionin sowie ihre Verwendung zur Schützung von den Hautzellen
Peptidomimetic methionine derivatives as well as their use in the protection of skin cells

(30) Priorité: 17.09.2010 FR 1003708
(43) Date de publication de la demande: 21.03.2012
(73) Titulaire: Exsymol S.A.M., 98000 Monaco (MC)
(72) Inventeur: Seguin, Marie-Christine, 98000 Monaco (MC)
(74) Mandataire: Macquet, Christophe

(56) Documents cités:
- EP-A1- 0 318 393
- WO-A2-2006/116353
- PICCIOLA G ET AL: "Derivati Della Metionina Ad Attivita Epatoprotettrice", FARMACO, EDIZIONE SCIENTIFICA, SOCIETA CHIMICA ITALIANA, PAVIA, IT, vol. 41, no. 10, 1 janvier 1986 (1986-01-01), pages 758-780, XP009149117, ISSN: 0430-0920

## Description

L'invention concerne une sélection de peptidomimétiques dérivés de l'acide aminé méthionine, et leur utilisation comme agent protecteur des mitochondries des cellules cutanées. L'invention concerne également des compositions destinées à prévenir ou lutter contre les désordres cutanés associés à un dysfonctionnement mitochondrial.

Organite cytoplasmique présent dans les cellules végétales et animales, la mitochondrie joue un rôle primordial pour ces dernières. En effet, les mitochondries sont annoncées être « les centrales énergétiques de nos cellules », avec la production et la mise en réserve d'adénosine triphosphate (ATP), composante énergétique universelle nécessaire au fonctionnement de toute cellule eucaryote.

Le comburant, nécessaire pour une telle production via un ensemble de réactions chimiques d'oxydo-réduction communément appelé "la chaîne respiratoire mitochondriale", est l'oxygène. L'oxygène est donc indispensable au fonctionnement cellulaire, mais paradoxalement, il est aussi la source initiale et parallèle d'espèces réactives appelées "espèces réactives dérivées de l'oxygène" (ERO en français, ROS en anglais), potentiellement toxiques pour les macromolécules biochimiques de la cellule (ADN, protéines, lipides, etc.).

Afin de neutraliser cette formation de ROS, toute cellule développe naturellement des systèmes de défense antioxydants, enzymatiques ou non, requérant pour leur mise en place de l'énergie, en premier lieu justement cet ATP issu de la chaîne respiratoire mitochondriale (Singh K.K., FEMS Yeast Res., 2004, vol.5, pp.127-132). Or, lorsqu'un déséquilibre entre production et élimination des ROS par les systèmes de défense intracellulaires survient au profit de quantités devenues trop intenses, le stress oxydant résultant engendre alors progressivement un déclin des fonctions mitochondriales, exprimé notamment par un dérèglement du flux d'électrons producteur d'énergie et la formation de ROS à l'intérieur même de la mitochondrie, appelés "ROS intra-mitochondriaux" (Jones D.P., Chemico Biological Comm., 2006, vol.163, pp.38-53). Un tel déséquilibre et déficit d'énergie au niveau de la cellule compromettent la capacité de celle-ci à s'adapter aux stress physiologiques auxquels elle est exposée, contribuant *in fine* au phénomène de vieillissement cellulaire (Trifunovic A. et al., J. of Internal Médecine, vol.263, pp.167-178). Il est établi aussi que ces dérégulations d'origine mitochondriale sont clairement impliquées dans la genèse d'un large spectre de pathologies ou désordres tissulaires (Barlow-Stewart K., The Australasian Genetics Ressource Book, 2007, pp.1-3 et références citées).

Source de ROS intracellulaire, il reste que la mitochondrie en est surtout la cible principale. En conséquence et afin de préserver au mieux l'intégrité fonctionnelle et/ou structurale de toute mitochondrie, l'une des préoccupations actuelles de nombre de chercheurs est de tenter de réduire le métabolisme oxydatif d'origine mitochondriale et de stimuler ou protéger les fonctions de la mitochondrie.

Dans l'état de la technique affiché ces dernières années avec de mêmes objectifs, les stratégies et systèmes retenus se sont souvent partagés entre :
- la sélection d'antioxydants puissants porteurs de un ou plusieurs motifs thiols ou phénols, et retrouvés à l'état naturel dans les règnes animal ou végétal, tels l'acide thioctique plus connu sous le nom d'acide alpha-lipoïque, aux bénéfices démontrés contre le vieillissement mitochondrial (Palaniappan A.R. et al., Neurochem. Res., 2007, vol.32, pp.1552-8), ou encore l'ergothionéine, dérivé thiourée annoncé protéger les membranes des mitochondries de mammifères (brevet US 6479533)
- l'utilisation de certaines molécules ou protéines antioxydantes conventionnelles, modifiées cependant par l'addition/greffage de groupements ou séquences (cation hydrophobe, etc.) à forte affinité pour la mitochondrie et pour une plus grande accumulation dans celle-ci (Kagan V.E. et al., Adv. Drug Delivery Rev., 2009, vol.61, pp.1375-85),
- le développement de petits peptides annoncés "cell-permeable" alternant des résidus aminoacides aromatiques et basiques, en raison de leur capacité à pénétrer la membrane interne mitochondriale et y exprimer ensuite des propriétés de cyto- et mitoprotection (Szeto H.H., Antioxidants & Redox Signaling, 2008, vol.3, pp.1-15).

La présente invention a été développée dans un même contexte d'identification de nouveaux produits ou nouvelles préparations pour répondre à la demande générale d'« améliorer le fonctionnement des mitochondries » (B. Lacroix, Nutranews, Avril 2008), à des fins toutefois cosmétiques et/ou dermatologiques avec l'administration de ces produits ou préparations principalement par voie topique cutanée et avec les objectifs concomitants suivants :
- préserver/restaurer une activité métabolique efficace des mitochondries des cellules de la peau lorsque ces dernières sont affectées physiologiquement ;
- présenter une biodisponibilité acceptable dans les couches profondes de la peau. D'une part, il est admis qu'il s'agit là d'un prérequis essentiel pour une protection in vivo des mitochondries des cellules cutanées. D'autre part, il est utile d'éviter une métabolisation trop précoce dans les couches supérieures de la peau avant même de pouvoir agir sur les cellules épidermiques et dermiques ;
- s'opposer aux effets délétères d'espèces réactives dérivées de l'oxygène vis-à-vis de ces mêmes cellules, tant des ROS de formation intra-mitochondriale que d'origine extracellulaire (rayonnements ultra-violets, pollution, toxines environnementales oxydantes, etc.).

Pour atteindre ces objectifs, la demanderesse s'est intéressée à des composés thioéthers pour lesquels il est attribué une capacité à désactiver ("quenching") divers états excités de l'oxygène, O₂^{°-} et ¹O₂ par exemple (Cohen S.G. et L., J. Am. Chem. Soc., 1975, vol.97, pp.5633-5634 et références citées).

Elle s'est plus particulièrement intéressée à l'acide alpha-aminé soufré méthionine dans lequel l'atome de soufre participe à une même fonction thioéther (S-CH₃), malgré certaines caractéristiques attachées à la méthionine contraires aux finalités recherchées : odeur reconnue aux dérivés thioorganiques peu appropriée avec une utilisation cosmétique/dermatologique, pouvoir de pénétration dans la peau faible, et surtout la mise en évidence de ROS intra-mitochondriaux et dommages oxydatifs de l'ADN mitochondrial générés par une supplémentation alimentaire de méthionine (Caro P. et al., Rev. Esp. Geriatr. Gerontol., 2009, vol.44, pp.194-199 ; Sanz A. et al., FASEB J., 2006, vol.20, pp.1064-1073). En outre, il est redouté pour les peptides de type oligométhionine (diméthionine, triméthionine, etc.) une sensibilité trop grande vis-à-vis de protéases cutanées.

C'est pourquoi en définitive la demanderesse a ensuite orienté ses recherches vers la synthèse de peptidomimétiques dérivés de la méthionine.

Ainsi, à l'issue d'une recherche structure-activité, le choix de la demanderesse s'est arrêté sur un panel limité de composés originaux dérivés de la méthionine, au regard de leur réponse avantageuse à la combinaison de critères susmentionnés et sans les inconvénients affichés par la méthionine ou les oligométhionines, réponse avantageuse illustrée par :
- une excellente capacité à maintenir l'activité métabolique de cellules cutanées exposées à un stress, reflétée par une préservation de la production des niveaux d'ATP [cf. test 1 ci-après] ;
- une absorption cutanée tout à fait favorable, allant même au-delà du stratum corneum, révélée par l'obtention d'une valeur logarithmique de son coefficient de perméabilité ("Log Kp") comparable à celle obtenue pour des composés perméants tels la caféine [cf. test 2 ci-après] ;
- une capacité à réduire le stress oxydant mitochondrial, ainsi qu'une capacité à moduler la masse mitochondriale (appelée "biogénèse mitochondriale") survenant en réponse à de mêmes conditions de stress [cf. test 3 ci-après] ;
- une cytoprotection élevée, exprimée en particulier sur une lignée cellulaire de fibroblastes "V79" [cf. test 4 ci-après] dont la caractéristique est d'être sensible au peroxyde d'hydrogène H₂O₂ à l'origine de désordres dans la chaîne respiratoire mitochondriale (Tatsumi T. et al., Basic Res. Cardiol., 1993, vol.88, pp.199-211) ;
- un fort profil antioxydant traduit par une capacité de piégeage d'espèces oxydantes connues pour altérer les mitochondries (radical hydroxyle OH°, ions péroxynitrites ONOO-, oxygène singulet ¹O₂), similaire à celle d'antioxydants de référence tels l'acide ascorbique ou le Trolox^{TM} [cf. tests 5 et 6 ci-après] ;
- une faible odeur dégagée par ces dérivés de méthionine.

L'invention a donc pour premier objet une famille de peptidomimétiques dérivés de la méthionine, caractérisée en ce qu'elle est représentée par la formule générale (I) suivants :

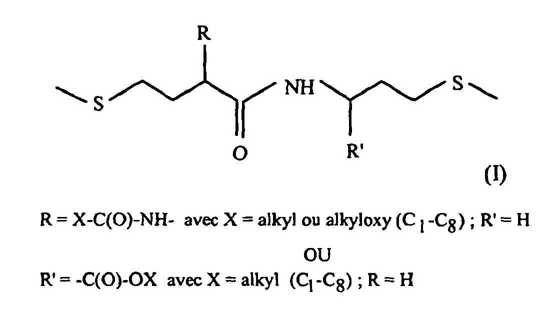

Suivant un mode de réalisation préféré de l'invention, la formule (I) est limitée à la formule (II) ci-après où le radical R' est exclusivement un atome d'hydrogène et R est un radical où X est de type alkyl ou alkyloxy avec une longueur de chaîne hydrocarbonée linéaire ou ramifiée comprenant de un à quatre atomes de carbone (C₁-C₄) :

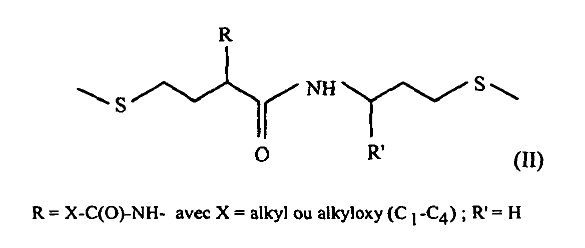

Plus avantageusement, X dans la formule (II) susmentionnée est de type alkyl avec une longueur de chaîne hydrocarbonée linéaire ou ramifiée comprenant de un à quatre atomes de carbone (C₁-C₄).

A titre d'exemples non limitatifs de composés de formule (II), on peut citer notamment les composés suivants :
- N-acétyl-(DL)-méthionyl-4-(méthylthio)propylamine
- N-propionyl-(DL)-méthionyl-4-(méthylthio)propylamine
- N-pentanoyl-(DL)-méthionyl-4-(méthylthio)propylamine
- N-t-butyloxy-(DL)-méthionyl-4-(méthylthio)propylamine

Suivant un mode de réalisation encore plus avantageux de l'invention, les formules (I) et (II) susmentionnées visent tout particulièrement le composé N-acétyl-(DL)-méthionyl-4-(méthylthio)propylamine (R = CH₃-C(O)-NH- et R¹ = H).

Selon un deuxième aspect, l'invention s'étend également à une composition, de préférence à usage cosmétique ou dermatologique, destinée à prévenir ou lutter contre les désordres cutanés associés à un dysfonctionnement mitochondrial, comprenant, en association avec tout adjuvant physiologiquement compatible avec la peau, à titre d'ingrédient actif principal, un peptidomimétique dérivé de la méthionine de formule générale (I) tel que défini ci-avant.

Dans le cadre de la présente invention, on entend par "ingrédient actif principal" une substance active capable de limiter les altérations fonctionnelles ou structurales des mitochondries de cellules cutanées soumises à un stress physico-chimique ou environnemental, par un processus de protection renforcée des mitochondries et/ou de stimulation de certaines de leurs fonctions essentielles, telles le métabolisme cellulaire énergétique.

Tout particulièrement, ladite composition est destinée à protéger la peau d'un stress induit par les radiations ultra-violettes (stress UV-induit), et le dérivé de la méthionine est de formule (II), plus particulièrement encore le composé N-acétyl-(DL)-méthionyl-4-(méthylthio)propylamine.

Avantageusement, la quantité dudit dérivé de formule générale (I) dans la composition ci-dessus est comprise entre 0,1 et 10 % en poids par rapport au poids total de la composition, de préférence entre 0,3 % et 3 % en poids.

Les compositions selon l'invention, de préférence à usage cosmétique ou dermatologique, sont adaptées à une administration par voie topique cutanée, présentées sous toutes les formes normalement utilisées pour une telle administration. De manière avantageuse, elles peuvent se présenter sous la forme d'une poudre, d'une émulsion, d'une microémulsion, d'une nanoémulsion, d'une suspension, d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Elles peuvent aussi être formulées pour une administration par voie orale (administration per os), sous forme par exemple de comprimés, gélules, capsules, cachets, ampoules, sirop, gouttes.

On peut citer, à titre d'exemple d'adjuvant physiologiquement compatible avec la peau, un composé choisi parmi les huiles, les cires, les élastomères de silicone, les tensioactifs, les cotensioactifs, les épaississants et/ou gélifiants, les humectants, les émollients, les filtres organiques, les filtres inorganiques, les boosters de filtres et les photostabilisants, les conservateurs, les colorants, les charges, les nacres, les agents matifiants, les agents tenseurs, les séquestrants, les parfums, et leurs mélanges.

De tels exemples, qui peuvent être présents dans la composition dans une teneur de l'ordre de 0,01 à 20% par rapport au poids total de la composition, sont notamment cités dans le dictionnaire "International Cosmetic Ingredient Dictionary and Handbook" (13ème Edition, 2010) publié par le "Personnal Care Product Council (PCPC, ex-CTFA)" de l'Association cosmétique américaine, et peuvent être (sans que cette liste ne soit limitative): des huiles de silicone, des huiles synthétiques ou d'origine naturelle, des hydrocarbures linéaires ou ramifiés, des esters et éthers de synthèse, des cires hydrocarbonées, des tensioactifs émulsionnants, des alcool gras linéaires ou ramifiés, des homo- et copolymères réticulés ou non, des gommes, des dérivés cellulosiques, des alginates, des polyols, sucres, glycosaminoglycanes et autres acides aminés, des charges minérales ou organiques, des protéines végétales, des polysaccharides, et leurs mélanges.

Les compositions selon l'invention peuvent en outre comprendre des actifs additionnels, de manière telle que l'effet intrinsèquement attaché aux compositions selon l'invention ne soit pas altéré par l'addition envisagée, en particulier au moins un actif choisi parmi les agents stimulant la production de facteurs de croissance, les agents anti-glycation ou déglycants, les agents augmentant la synthèse de collagène ou prévenant sa dégradation, les agents augmentant la synthèse d'élastine ou prévenant sa dégradation, les agents augmentant la synthèse de glycosaminoglycanes ou de protéoglycanes ou prévenant leur dégradation, les agents augmentant la prolifération ou la différenciation des kératinocytes, les agents augmentant la prolifération des fibroblastes, les agents dépigmentants, anti-pigmentants ou pro-pigmentants, les agents antioxydants ou anti-radicalaires ou anti-pollution, les agents stimulant l'hydratation et/ou protégeant la fonction barrière de la peau, les agents augmentant la synthèse de lipides épidermiques, les agents stimulant la lipolyse, inhibant la lipogenèse et/ou inhibant la différenciation des adipocytes, les agents drainants ou détoxifiants, les agents anti-inflammatoires, les agents accélérateurs de pénétration, les agents desquamants, les agents apaisants et/ou anti-irritants, les agents astringents, les agents agissant sur la microcirculation, les agents agissant sur le métabolisme des cellules, et leurs mélanges, et sans que cette liste ne soit limitative.

Des exemples de tels actifs additionnels peuvent être présents dans la composition dans une teneur de l'ordre de 0,001 à environ 10% par rapport au poids total de la composition, et peuvent être notamment choisis parmi des extraits de plante, des dérivés de silicium, des extraits de levure et d'algues, des hydrolysats de protéines végétales, des oligopeptides acylés ou non, des extraits de café, la carcinine et ses dérivés, la carnosine et ses dérivés, la N-acétylcystéine et ses dérivés, des vitamines hydrosolubles telles que les vitamines B1, B2, B3, B6, B12, C, H, des vitamines liposolubles telles que les vitamines A, D2, D3, E et carotène, l'urée et ses dérivés, la taurine et ses dérivés, des polyphénols, des oligo- et polysaccharides, les acides lactique, glycolique, citrique et salicylique et leurs esters ou sels, et leurs mélanges.

Un autre objet de l'invention concerne également l'utilisation d'un peptidomimétique dérivé de la méthionine comme agent cosmétique destiné à protéger et/ou stimuler les mitochondries des cellules cutanées, ledit dérivé étant de formule générale (III) suivante : 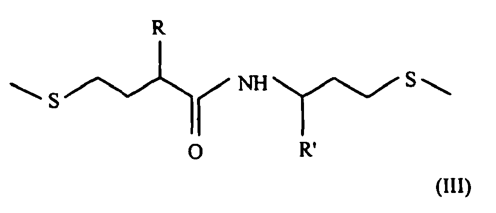 R = X-C(O)-NH- avec X = alkyl ou alkyloxy (C₁-C₈) ; R^{'} = H OU -C(O)R" avec R" = Oalkyl (C ₁-C₄), NH₂
OU
R' = -C(O)-OX avec X = alkyl (C₁-C₈); R = H

Suivant un mode de réalisation préféré de l'invention, ledit dérivé est de formule générale (III) suivants : 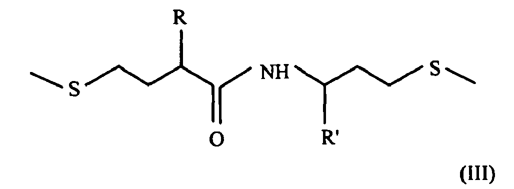 R = X-C(O)-NH- avec X = alkyl ou alkyloxy (C₁-C₈); R' = H OU -C(O)R" avec R" = Oalkyl (C₁-C₄), NH₂

Suivant un mode de réalisation avantageux de l'invention, il est cherché, avec l'utilisation susmentionnée dudit dérivé, à prévenir ou à lutter contre les signes cutanés du vieillissement, préférentiellement contre les signes cutanés du vieillissement photo-induit, en particulier contre les dommages causés à la peau par les rayonnements ultra-violets.

Un dernier objet de l'invention concerne un peptidomimétique dérivé de la méthionine pour son utilisation dans une composition dermatologique pour le traitement des signes cutanés du vieillissement, préférentiellement contre les dommages causés à la peau par les radiations ultra-violettes, ledit dérivé étant de formule générale (III) suivants : 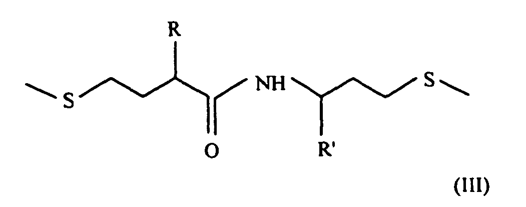 R = X-C(O)-NH- avec X = alkyl ou alkyloxy (C₁-C₈); R' = H ou -C(O)R" avec R" = Oalkyl (C₁-C₄), NH₂
OU
R' = -C(O)-OX avec X = alkyl (C₁-C₈); R = H

Suivant un mode de réalisation préféré de l'invention, ledit dérivé est de formule générale (III) suivants : 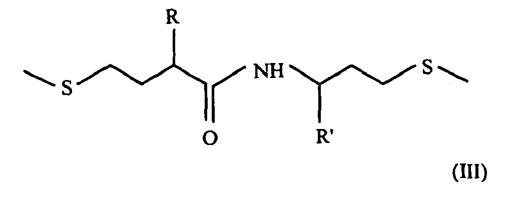 R = X-C(O)-NH- avec X = alkyl ou alkyloxy (C₁-C₈) ; R' = H OU -C(O)R" avec R" = Oalkyl (C₁-C₄), NH₂

A titre d'exemples non limitatifs de composés de formule (III), on peut citer notamment les composés suivants :
- N-acétyl-(DL)-méthionyl-4-(méthylthio)propylamine
- N-propionyl-(DL)-méthionyl-4-(méthylthio)propylamine
- N-pentanoyl-(DL)-méthionyl-4-(méthylthio)propylamine
- N-t-butyloxy-(DL)-méthionyl-4-(méthylthio)propylamine
- N-4-(méthylthio)-butyryl-L-méthionine méthylester
- N-acétyl-(DL)-méthionyl-méthionine éthylester

Dans les deux derniers objets de l'invention tels que ci-dessus, il est préférentiellement retenu un dérivé de formule générale (III) tel que R est limité à un radical où X est de type alkyl comprenant de un à quatre atomes de carbone (C₁-C₄) et R' est exclusivement un atome d'hydrogène.

De préférence encore, il est tout particulièrement sélectionné le composé N-acétyl-(DL)-méthionyl-4-(méthylthio)propylamine.

### Exemple 1 :

A titre d'illustration, il est mentionné ci-après des exemples de formulation de composition selon l'invention, contenant un dérivé de formule générale (I) précitée

### Formule A (crème)

:

| | |
|---|---|
| N-acétyl-(DL)-mêthionyl-4-(mêthylthio)propylamine | 1 % |
| Polyisobutène hydrogéné | 7 % |
| Myristate d'isobutyle | 3 % |
| Palmitate de cétyle | 7 % |
| Monostéarate d'éthylène glycol | 5 % |
| Laurate sorbitan | 2 % |
| Polysorbate 20 | 2 % |
| Carbomer (copolymère d'acrylate/acrylamide & huile minérale) | 0,3 % |
| Phénoxyéthanol | 0,5 % |
| Benzoate de sodium | 0,2 % |
| Eau | qsp 100 % |

### Formule B (gel)

| | |
|---|---|
| N-t-butyloxy-(DL)-méthionyl-4-(méthylthio)propylamine | 3 % |
| Carbomer (copolymère d'acrylate/acrylamide & huile minérale) | 1,5 % |
| Benzoate de sodium | 0,2 % |
| Acide sorbique | 1 % |
| 1,3-butanediol | 10 % |
| Glycérine | 5 % |
| Soude | 0,13 % |
| Phénoxyéthanol | 0,9 % |
| Eau | qsp 100 % |

### Formule C (gélule, administration per os)

N-acétyl-(DL)-méthionyl-4-(méthylthio)propylamine (200 mg poudre/gélule)
OU
N-4-(méthylthio)-butyryl-L-méthionine méthylester (200 mg poudre/gélule)

Excipients qsp 1 gélule en gélatine : cellulose microcristalline, stéarate de magnésium

### Exemple 2 :

L'invention est illustrée ci-après, à titre purement indicatif par les tests suivants évoqués plus haut dans la description de l'invention (tests 1 à 6). Il est aussi à signaler que des études in vivo menées sur l'Homme sont en cours de réalisation et que les premiers résultats sont également en mesure d'illustrer l'utilisation de certains peptidomimétiques dérivés de la méthionine aux fins de la présente invention.

### Test 1 mise en évidence de la capacité de peptidomimétiques dérivés de la méthionine à restaurer les niveaux d'ATP cellulaire sur des cellules objets d'un stress (H₂O₂) :

L'étude expérimentale a été réalisée sur une lignée cellulaire de fibroblastes de type "V79" et ensemencée dans une plaque 96 puits à raison de 5000 cellules par puits dans 100 µl de milieu de culture (contenant 10% de sérum de veau foetal). Celui-ci est ensuite remplacé dans chacun des puits par 100 µl de milieu contenant des peptidomimétiques selon l'invention, à la concentration de 7,5 mM ou 10 mM. Après 2 heures d'incubation, le milieu est retiré sur l'ensemble des puits, puis les cellules sont soumises à un état de stress avec l'ajout d'un milieu contenant 50 µl de peroxyde d'hydrogène H₂O₂ (4 ppm). Après 8 nouvelles heures d'incubation, la concentration moyenne en ATP est mesurée par luminométrie en présence de luciférine (kit "ATPlite 1step") et à l'aide d'une courbe étalon préparée à partir d'ATP hautement purifié.

Les résultats, rassemblant les valeurs moyennes obtenues à partir de trois expérimentations indépendantes, sont présentés dans le tableau 1 ci-après, en comparaison à un contrôle.

**Tableau 1**

| **Composé** | **ATP (X10⁻⁷M)** |
|---|---|
| contrôle | 4,42 |
| contrôle + H₂O₂ | 0,4 |
| N-acétyl-(DL)-mëthionyl-4-(méthylthio)propylamine 7,5 mM | 5,91 |
| N-acétyl-(DL)-méthionyl-4-(méthylthio)propylamine 7,5 mM + H₂O₂ | 4,83 |
| N-propionyl-(DL)-méthionyl-4-(méthylthio)propylamine 10 mM | 5,28 |
| N-propionyl-(DL)-méthionyl-4-(méthylthio)propylamine 10 mM + H₂O₂ | 4,49 |

La chute massive de la quantité d'ATP observée pour le contrôle est très largement prévenue en présence des composés selon l'invention, démontrant ainsi sa capacité à restaurer la production d'ATP au sein de cellules soumises à un stress.

### Test 2 étude d'absorption percutanée sur explants de peau humaine de peptidomimétiques dérivés de la méthionine :

Les valeurs de coefficient de perméabilité (Kp) ont été obtenues sur une peau humaine issue d'une plastie abdominale préalablement congelée, conformément au protocole décrit dans la ligne directrice n° 428 de l'OCDE sur la conduite d'études d'absorption cutanée.

Expérimentalement, les explants de peau ont été placés sur cellule de Frantz en diffusion passive, pilotés par le système "MicroettePlus Hanson Research", et les produits testés sont appliqués de façon non occlusive. Suite à cette mise en place de la peau sur les cellules, 500 µl du peptidomimétique selon l'invention (solution à 1%) ont été déposés dans chacune des cellules et pour un temps de contact total de 24 heures. Au terme de celles-ci, le produit restant est prélevé et la surface de la peau rincée pour extraction et mesure de la quantité absorbée, puis pour la détermination du coefficient de perméabilité exprimé sous forme logarithmique (Log Kp).

Les résultats sont rassemblés dans le tableau 2 ci-après.

**Tableau 2**

| **Composé** | **Kp (cm.h⁻¹)** | **Log Kp** |
|---|---|---|
| caféine | 1.10⁻⁴ ** | - 4,0 |
| N-acétyl-(DL)-méthionyl-4-méthylthio)propylamine | 1, 09.10⁻⁴ | - 3,96 |
| N-propionyl-(DL)-méthionyl-4-(méthylthio)propylamine | 9,7.10⁻⁵ | -4,0 |
| N-pentanoyl-(DL)-méthionyl-4-(méthylthio)propylamine | 3,7.10⁻⁴ *** | - 3,43 |
| N-t-butyloxy-(DL)-méthionyl-4-(méthylthio)propylamine | 4,6.10⁻⁴ *** | - 3,34 |
| N-4-(méthylthio)-butyryl-L-méthionine méthylester | 2.10-4 *** | - 3,71 |
| N-acétyl-(DL)-mëthionyl-méthionine éthylester | 1,64.10⁻⁴ *** | - 3,78 |

| | | |
|---|---|---|
| ** : Mitragotri S., J. Controlled Release, (2003), vol.86, pp.69-92 *** : selon logiciel prédictif "ChemDraw ultra version 11.0", fournisseur : CambridgeSoft Ltd | | |

Le coefficient de perméabilité obtenu pour les composés selon l'invention est comparable à celui obtenu pour la caféine, reconnue perméante trans-stratum corneum.

### Test 3 mise en évidence de l'effet protecteur du composé N-acétyl-(DL)-méthionyl-4-(méthylthio)propylamine sur le stress oxydant mitochondrial et sur la biogénèse mitochondriale

Principes : les mesures de stress oxydant intracellulaire mitochondrial et la détection de la biogénèse mitochondriale ont été réalisées par cytométrie de flux à l'aide des marqueurs fluorochromes suivants :
- la sonde fluorescente rouge "MitoSOX^{™} Red" ci-après désignée "MitoSOX" (détection λ = 580 nm ; fournisseur : Invitrogen) permet de détecter spécifiquement l'anion supéroxyde O₂°⁻ présent dans la mitochondrie (en raison de sa forte affinité pour celle-ci),
- la sonde fluorescente verte "MitoTracker^{®} Green FM" ci-après désignée "Mitogreen" (détection a λ = 516 nm ; fournisseur : Invitrogen) permet de quantifier la masse mitochondriale (ou nombre relatif de mitochondries par cellule).

Expérimentalement, les essais ont été réalisés sur une lignée fibroblastique adhérente de hamster "V79", maintenue par repiquage dans du milieu de culture complet "EMEM" (avec 10% de sérum de veau foetal) en atmosphère humide à 37 °C et 5 % de CO₂. Les cellules V79 sont ensemencées puis incubées 24 heures dans des plaques 6 puits à raison de 2,5.10⁵ cellules par puits dans 3 ml du même milieu de culture, puis soumises à un état de stress pendant 1h30 avec l'ajout d'un milieu contenant du peroxyde d'hydrogène H₂O₂ (15 ppm).

Après trypsination, les cellules sont incubées à 37°C pendant 15 minutes dans 1 ml de milieu de culture complet "EMEM" et contenant les marqueurs susmentionnés "MitoSOX" (à la concentration finale de 5 µM) et "Mitogreen" (à la concentration finale de 200 nM), pour détection respective :
- du stress oxydant mitochondrial (exprimé en % de cellules positives),
- et de la masse mitochondriale par cellule (exprimée en % de MFI ("Mean Fluorescence Intensity") par rapport au contrôle).
du stress oxydant mitochondrial (exprimé en % de cellules positives),

Les résultats, rassemblant les valeurs moyennes obtenues à partir de quatre expérimentations indépendantes, sont présentés dans les tableaux 3a et 3b ci-après, en comparaison avec ceux obtenus pour la N-acétyl-cystéine choisie comme agent protecteur de référence à la concentration de 5 mM.

**Tableau 3a**

| | **% de cellules positives au MitoSOX** |
|---|---|
| contrôle | 18 |
| contrôle + H₂O₂ | 85,7 |
| N-acétyl-cystéine 5 ppm + H₂O₂ | 15,2 |
| N-acétyl-(DL)-méthionyl-4-(méthylthio)propylamine 7,5 mM + H₂O₂ | 40,3 |

**Tableau 3b**

| | **MFI (%contrôle)** |
|---|---|
| contrôle | 100 |
| contrôle + H₂O₂ | 177,3 |
| N-acétyl-cystéine 5 ppm + H₂O₂ | 74,4 |
| N-acétyl-(DL)-méthionyl-4-(méthylthio)propylamine 7,5 mM + H₂O₂ | 123,5 |
| N-acétyl-(DL)-méthionyl-4-(méthylthio)propylamine 10 mM + H₂O₂ | 127,5 |

Dans les deux cas, les résultats soulignent pour le composé N-acétyl-(DL)-méthionyl-4-(méthylthio)propylamine une capacité à minorer l'effet du stress intramitochondrial.

### Test 4 mise en évidence de l'effet cytoprotecteur des peptidomimétiques N-acétyl-(DL)-méthionyl-4-(méthylthio)propylamine et N-t-butyloxy-(DL)-méthionyl-4-(mêthylthio)propylamine :

L'étude expérimentale a été réalisée sur une lignée fibroblastique adhérente de hamster "V79", maintenue en atmosphère humide à 37 °C et 5 % de CO₂, puis ensemencée dans des plaques 96 puits à raison de 0,5.10⁴ cellules par puits dans 0,2 ml du milieu de culture complet "EMEM" (avec 10% de sérum de veau foetal). Les cellules sont alors soumises 24 heures à un état de stress, toxique, avec le remplacement du milieu de culture par un milieu contenant du peroxyde d'hydrogène H₂O₂ (4 ppm), milieu auquel est ajouté simultanément le composé selon l'invention. Après élimination du milieu, la viabilité cellulaire des fibroblastes est mesurée via la "méthode au MTT" ou bromure de 3-(4,5-diméthylthiazol-2-yl)-2,5-diphenyltétrazolium (solution à 500 µg/ml) et par spectrophotométrie (absorbance à 540 nm).

Les résultats, rassemblant les valeurs moyennes obtenues à partir de trois expérimentations indépendantes, sont présentés dans le tableau 4 ci-après, à nouveau en comparaison avec ceux obtenus pour la N-acétyl-cystéine à 5 mM choisie comme cytoprotecteur de référence (restauration totale d'une viabilité cellulaire).

**Tableau 4**

| | **% viabilité cellulaire** |
|---|---|
| contrôle | 100 |
| contrôle + H₂O₂ | 54,8 |
| N-acétyl-cystéine 5 mM | 103,8 |
| N-acétyl-(DL)-méthionyl-4-(méthylthio)propylamine 7,5 mM + H₂O₂ | 83,5 |
| N-acétyl-(DL)-méthionyl-4-(méthylthio)propylamine 10 mM + H₂O₂ | 91, 9 |
| N-t-butyloxy-(DL)-méthionyl-4-(méthylthio)propylamine 7,5mM + H₂O₂ | 70,1 |
| N-t-butyloxy-(DL)-mëthionyl-4-(méthylthio)propylamine 10 mM + H₂O₂ | 77,3 |

Les résultats du tableau 4 soulignent, pour les composés selon l'invention, une viabilité cellulaire dose-dépendante et une capacité à protéger les cellules d'un stress cytotoxique.

### Test 5 mise en évidence de l'effet antioxydant des peptidomimétiques N-acétyl-(DL)-méthionyl-4-(méthylthio)propylamine et N-propionyl-(DL)-méthionyl-4-(méthylthio)propylamine vis-à-vis du radical hydroxyle

La méthode, décrite par Rehman A. et coll. (British J. Pharmacol. (1997), vol. 122, pp. 1702-1706), est utilisée pour la détermination de la constante de vitesse de piégeage du radical hydroxyle [Ks(OH°)], le peptidomimétique selon l'invention étant comparé à deux antioxydants de référence, le mannitol et l'acide ascorbique (vitamine C).

Expérimentalement, la substance testée est dissoute dans un milieu tamponné à pH 7,4 auquel est ajouté un milieu générateur de OH° (système ascorbate/fer/EDTA) en présence de désoxyribose. Après une heure d'incubation à 37°C, la réaction est arrêtée à l'aide d'acide trichloroacétique. Après révélation colorimétrique par l'acide thiobarbiturique, il est mesuré l'absorbance à 532 nm pour différentes concentrations, puis calculé le Ks(OH°) relatif à chacune des substances.

Les résultats sont reportés dans le tableau 5 ci-après.

**Tableau 5**

| **Composé** | **Ks(OH°) (10⁹.m⁻¹.S⁻¹)** |
|---|---|
| acide ascorbique | 10,1** |
| mannitol | 1,6 / 1,9*** |
| N-acétyl-(DL)-méthionyl-4-(méthylthio)propylamine | 6,1 |
| N-propionyl-(DL)-méthionyl-4-(méthylthio)propylamine | 5,7 |

| | |
|---|---|
| ** : Cabelli D.E., J. Phys. Chem. (1983), vol.87, pp.1809-1812] *** : Rehman A., British J. Pharmacol. (1997), vol.122, pp.1702-1706)] | |

Les résultats, rassemblant les valeurs moyennes obtenues à partir de trois expérimentations indépendantes, soulignent pour les peptidomimétiques selon l'invention une capacité à piéger le radical hydroxyle (OH) très largement supérieure à celle du mannitol, intermédiaire en comparaison avec celle de l'acide ascorbique.

### Test 6 mise en évidence de l'effet antioxydant du composé N-acétyl-(DL)-méthionyl-4-(méthylthio)propylamine vis-à-vis des ions péroxynitrites

La méthode dite "test du blanchiment du pyrogallol red (PR)", décrite par Nath V.B. et coll. (BBRC (2001), vol. 285, pp. 262-266), est utilisée pour la détermination de l'inhibition de l'oxydation du rouge de pyrogallol (PR) par des ions péroxynitrites (ONOO⁻), le composé N-acétyl-(DL)-méthionyl-4-(méthylthio)propylamine selon l'invention étant comparé à un antioxydant de référence, à savoir le "Trolox^{™}" ou acide 2-(6-hydroxy-2,5,7,8-tetraméthylchromane) carboxylique, équivalent hydrosoluble de la vitamine E.

Expérimentalement, dans une plaque 96 puits, à la substance testée dissoute dans un milieu tamponné à pH 7,0 auquel sont ajoutés 50 µM de PR et 25 µM d'ions péroxynitrites ONOO⁻ (en solution dans NaOH 0,1 M). Après 5 minutes, il est mesuré l'absorbance par spectrophotométrie à 540 nm, puis calculé l'IC50 (concentration inhibitrice de 50%) relatif à chacune des substances. Les résultats sont reportés dans le tableau 6 ci-après.

**Tableau 6**

| **Composé** | **IC₅₀ (mM)** |
|---|---|
| Trolox | 0,083 |
| N-acétyl- (DL) -méthionyl-4-(méthylthio)propylamine | 1,012 |

Moins actif que l'antioxydant de référence, l'inhibition de l'oxydation du PR par les ions péroxynitrites reste toutefois clairement observée pour le composé selon l'invention.

## Revendications

1. Peptidomimétique dérivé de la méthionine, caractérisé en qu'il est représenté par la formule générale (II) suivante :

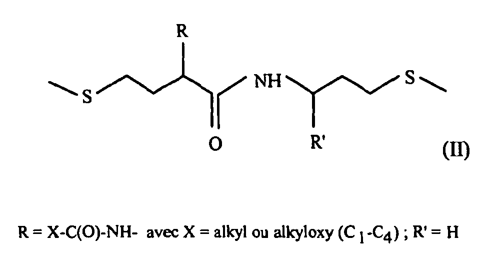

2. Peptidomimétique selon la revendication 1, **caractérisé en ce qu'**il s'agit du N-acétyl-(DL)-méthionyl-4-(méthylthio)propylamine.

3. Composition destinée à prévenir ou lutter contre les désordres cutanés associés à un dysfonctionnement mitochondrial, **caractérisée en ce qu'**elle comprend un peptidomimétique dérivé de la méthionine de formule générale (II) suivante :

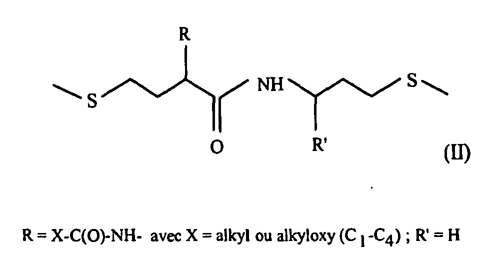

4. Composition selon la revendication 3, **caractérisé en ce que** ledit peptidomimétique est le N-acétyl-(DL)-méthionyl-4-(méthylthio)propylamine.

5. Composition selon l'une des revendications 3 et 4, **caractérisée en ce que** la quantité dudit peptidomimétique est comprise entre 0,1 et 10 % en poids par rapport au poids total de la composition.

6. Composition selon l'une quelconque des revendications 3 à 5, **caractérisée en ce qu'**elle est adaptée à une administration par voie topique cutanée, présentée sous la forme d'une poudre, d'une émulsion, d'une microémulsion, d'une nanoémulsion, d'une suspension, d'une lotion, d'une crème, d'un gel aqueux ou hydroalcoolique, d'une mousse, d'un sérum, d'une solution ou d'une dispersion pour aérosol, ou d'une dispersion de vésicules lipidiques.

7. Composition selon l'une quelconque des revendications 3 à 6, **caractérisée en ce qu'**elle comprend des actifs additionnels choisis parmi les agents stimulant la production de facteurs de croissance, les agents anti-glycation ou déglycants, les agents augmentant la synthèse de collagène ou prévenant sa dégradation, les agents augmentant la synthèse d'élastine ou prévenant sa dégradation, les agents augmentant la synthèse de glycosaminoglycanes ou de protéoglycanes ou prévenant leur dégradation, les agents augmentant la prolifération ou la différenciation des kératinocytes, les agents augmentant la prolifération des fibroblastes, les agents dépigmentants, anti-pigmentants ou pro-pigmentants, les agents antioxydants ou anti-radicalaires ou anti-pollution, les agents stimulant l'hydratation et/ou protégeant la fonction barrière de la peau, les agents augmentant la synthèse de lipides épidermiques, les agents stimulant la lipolyse, inhibant la lipogenèse et/ou inhibant la différenciation des adipocytes, les agents drainants ou détoxifiants, les agents anti-inflammatoires, les agents accélérateurs de pénétration, les agents desquamants, les agents apaisants et/ou anti-irritants, les agents astringents, les agents agissant sur la microcirculation, les agents agissant sur le métabolisme des cellules, et leurs mélanges.

8. Composition selon l'une quelconque des revendications 3 à 7, **caractérisé en ce qu'**elle est destinée à protéger la peau d'un stress "UV-induit".

9. Utilisation d'un peptidomimétique dérivé de la méthionine comme agent cosmétique destiné à protéger et/ou stimuler les mitochondries des cellules cutanées, ledit peptidomimétique étant de formule générale (III) suivante :

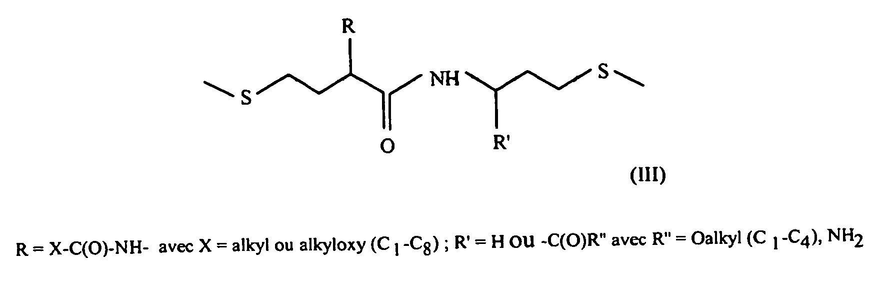

10. Peptidomimétique dérivé de la méthionine pour son utilisation dans une composition dermatologique pour le traitement des signes cutanés du vieillissement, ledit dérivé étant de formule générale (III) suivante :

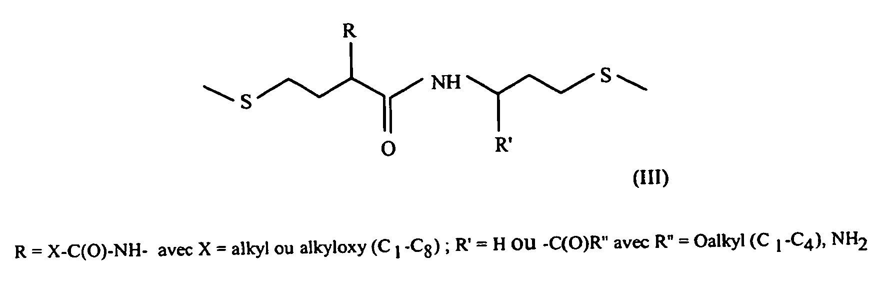

## Patentansprüche

1. Peptidomimetikum, abgeleitet von Methionin, **dadurch gekennzeichnet, dass** es von der folgenden allgemeinen Formel (II) dargestellt wird:

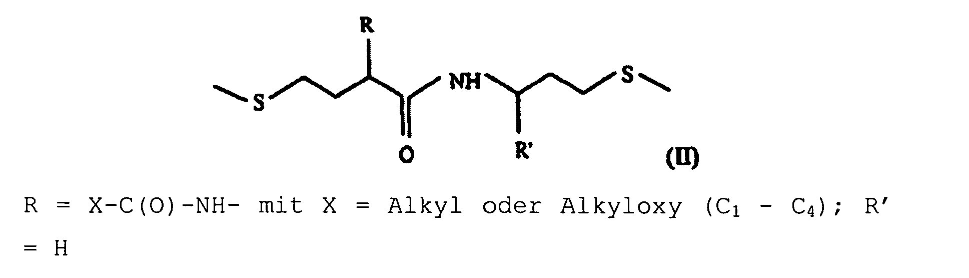

2. Peptidomimetikum nach Anspruch 1, **dadurch gekennzeichnet, dass** es sich um das N-acetyl-(DL)-methionyl-4-(methylthio)propylamin handelt.

3. Zusammensetzung, ausgelegt, um Hautstörungen vorzubeugen oder dagegen anzukämpfen, verbunden mit einer mitochondrialen Fehlfunktion, **dadurch gekennzeichnet, dass** sie ein Peptidomimetikum umfasst, abgeleitet von Methionin mit der folgenden allgemeinen Formel (II):

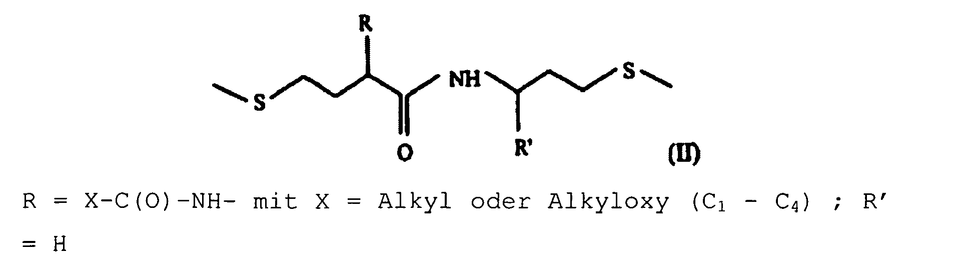

4. Zusammensetzung nach Anspruch 3, **dadurch gekennzeichnet, dass** das Peptidomimetikum das N-acetyl-(DL)-methionyl-4-(methylthio)propylamin ist.

5. Zusammensetzung nach einem der Ansprüche 3 und 4, **dadurch gekennzeichnet, dass** die Menge des Peptidomimetikums zwischen 0,1 und 10 Gew.% mit Bezug auf das Gesamtgewicht der Zusammensetzung liegt.

6. Verbindung nach einem der Ansprüche 3 bis 5, **dadurch gekennzeichnet, dass** sie für eine Verabreichung auf topischem kutanem Weg ausgelegt ist, präsentiert in der Form eines Puders, einer Emulsion, einer Mikroemulsion, einer Nanoemulsion, einer Suspension, einer Lotion, einer Creme, eines wässrigen oder wässrig-alkoholischen Gels, eines Schaums, eines Serums, einer Lösung oder einer Dispersion für Aerosol oder einer Dispersion von Lipidvesikeln.

7. Zusammensetzung nach einem der Ansprüche 3 bis 6, **dadurch gekennzeichnet, dass** sie zusätzliche Wirkstoffe umfasst, ausgewählt aus den Stoffen, die die Erzeugung von Wachstumsfaktoren stimulieren, den Glykierungsinhibitoren oder Deglykanten, .den Stoffen, die die Synthese von Kollagen erhöhen oder seinem Abbau vorbeugen, den Stoffen, die die Synthese von Elastin erhöhen oder seinem Abbau vorbeugen, den Stoffen, die die Synthese von Glycosaminoglycanen oder von Proteoglycanen erhöhen oder ihrem Abbau vorbeugen, den Stoffen, die die Proliferation oder die Differentiation der Keratinocyten erhöhen, den Stoffen, die die Proliferation der Fibroplasten erhöhen, den Depigmentierungsmitteln, Anti-Pigmentierungsmitteln oder Pro-Pigmentierungsmitteln, den Anti-Oxidation-, anti-radikalischen oder Anti-Verschmutzungsmittel, den Stoffen, die die Hydrierung stimulieren und/oder die Barrierefunktion der Haut schützen, den Stoffen, die die Synthese der epidermalen Lipide erhöhen, den Stoffen, die die Lipolyse stimulieren und die Lipogenese hemmen und/oder die Differentiation der Adipocyten verhindern, den Abtropf- oder Entgiftungsmittel, den entzündungshemmenden Mitteln, den die Eindringung beschleunigenden Mitteln, den Hautschuppen entfernenden Mitteln, den lindernd wirkenden und/oder reizvermeidenden Mitteln, den astringenten Mitteln, den Mitteln, die sich auf die Mikrozirkulation auswirken, den Mitteln, die sich auf den Stoffwechsel der Zellen auswirken, und ihren Mischungen.

8. Zusammensetzung nach einem der Ansprüche 3 bis 7, **dadurch gekennzeichnet, dass** sie dazu ausgelegt ist, die Haut vor einem "UV-induzierten" Stress zu schützen.

9. Verwendung eines Peptidomimetikums, abgeleitet von Methionin, als kosmetisches Mittel, das dazu ausgelegt ist, die Mitochondrien der Hauzellen zu schützen und/oder zu stimulieren, wobei das Peptidomimetikum die allgemeine Formel (III) aufweist:

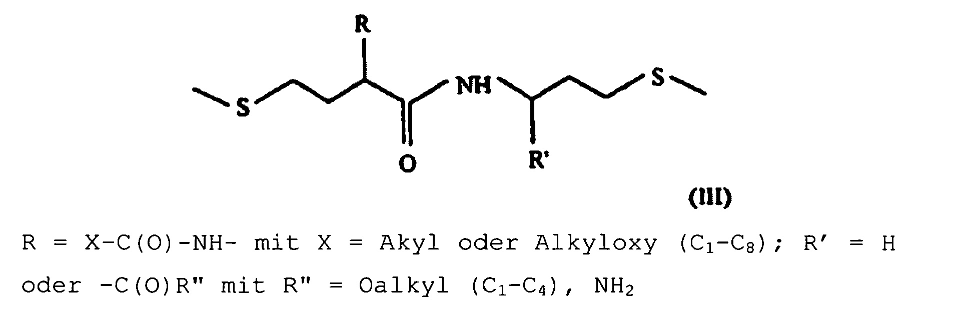

10. Peptidomimetikum, abgeleitet von Methionin, um in einer dermatologischen Zusammensetzung verwendet zu werden, zu Behandlung von altersbedingten Hautanzeichen, wobei das Derivat die folgende allgemeine Formel (III) aufweist:

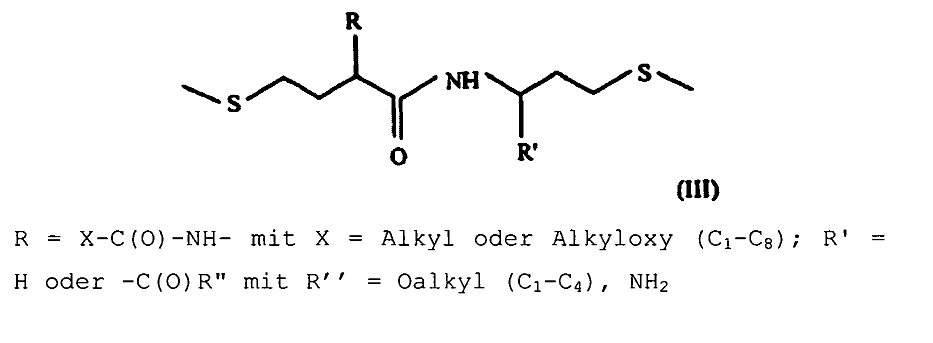

## Claims

1. Methionine-derived peptidomimetic, **characterized in that** it is represented by the following general formula (II) :

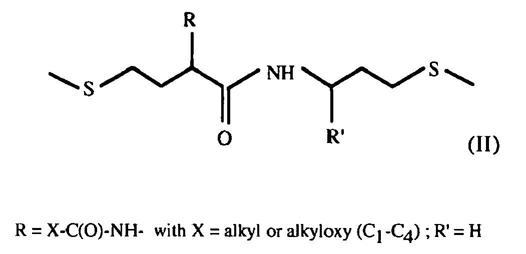

2. Peptidomimetic according to claim 1, **characterized in that** it is N-acetyl-(DL)-methionyl-4- (methylthio)propylamine.

3. Composition intended for preventing or fighting against skin disorders associated to a mitochondrial dysfunction, **characterized in that** it comprises a methionine-derived peptidomimetic according to the following general formula (II) :

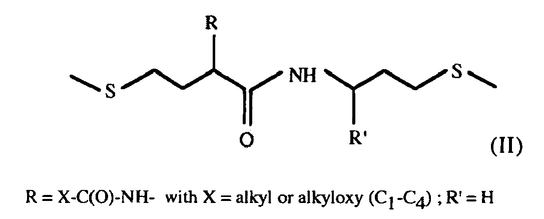

4. Composition according to claim 3, **characterized in that** said peptidomimetic is N-acetyl-(DL)-methionyl-4-(methylthio)propylamine.

5. Composition according to one of claims 3 and 4, **characterized in that** the amount of said peptidomimetic is between 0.1 and 10 % in weight in relation to the total weight of the composition.

6. Composition according to one of claims 3 to 5, **characterized in that** it is adapted to an administration by cutaneous topical route under the form of a powder, of an emulsion, of a microemulsion, of a nanoemulsion, of a suspension, of a lotion, of a cream, of an aqueous or hydroalcoholic gel, of a foam, of a serum, of a solution or a dispersion for spray, or of a lipidic vesicles' dispersion.

7. Composition according to one of claims 3 to 6, **characterized in that** it comprises additional active ingredients chosen among agents for stimulating production of growth factors, anti-glycation or deglycation agents, agents for increasing collagen synthesis or prevent its degradation, agents for increasing elastin synthesis or prevent its degradation, agents for increasing synthesis of glycosaminoglycans or proteoglycans or prevent their degradation, agents for increasing keratinocytes' proliferation or differentiation, agents for increasing fibroblasts' proliferation, bleaching, anti-pigmenting or pro-pigmenting agents, antioxidant or anti-radical or anti-pollution agents, agents for stimulating hydration and/or protecting skin barrier function, agents for increasing epidermal lipids' synthesis, agents for stimulating lipolysis, for inhibiting lipogenesis and/or inhibiting adipocytes' differentiation, draining or detoxifying agents, anti-inflammatory agents, penetration enhancer agents, pelling agents, soothing and/or anti-irritating agents, astringent agents, agents for acting on microcirculation, agents for acting on cell metabolism, and mixtures thereof.

8. Composition according to one of claims 3 to 7, **characterized in that** it is intended for protecting skin from a "UV-induced" stress.

9. Use of a methionine-derived peptidomimetic as a cosmetic agent intended for protecting and/or stimulating skin cells' mitochondria, said peptidomimetic having the following general formula (III) :

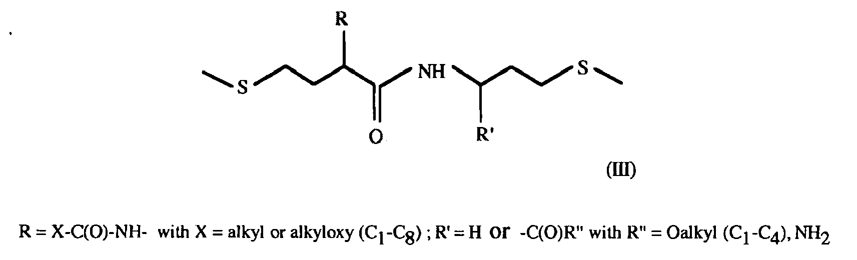

10. Methionine-derived peptidomimetic for its use in a dermatological composition intended for treating cutaneous signs of aging, said derivative having the following general formula (III) :
